## Europäisches Patentamt

## European Patent Office

## Office européen des brevets

(19)

(11) Numéro de publication: **0 194 192**
**B1**

(12)

# FASCICULE DE BREVET EUROPEEN

(45) Date de publication du fascicule du brevet:
**08.11.89**

(51) Int. Cl.⁴: **A 61 L 27/00**

(21) Numéro de dépôt: **86400375.1**

(22) Date de dépôt: **21.02.86**

(54) Matériau chirurgical composite absorbable, procédé de préparation, prothèse resorbable réalisée à partir d'un tel matériau, et utilisation d'une telle prothèse.

(30) Priorité: **22.02.85 FR 8502565**

(43) Date de publication de la demande:
**10.09.86 Bulletin 86/37**

(45) Mention de la délivrance du brevet:
**08.11.89 Bulletin 89/45**

(84) Etats contractants désignés:
**DE FR GB**

(56) Documents cités:
**EP-A- 0 011 528**
**DE-A- 2 152 142**
**GB-A- 1 008 193**

(73) Titulaire: **ETHNOR, 192, Avenue Charles de Gaulle,
F-92200 Neuilly sur Seine (FR)**

(72) Inventeur: **Bilweis, Joseph, 10 l'Orée de Marly,
F-78590 Noisy Le Roi (FR)**
Inventeur: **Gorham, Steve, Dr., c/o DEVERO LTD
Moodestburn, Chryston Glasgow, G 69 0JE (GB)**

(74) Mandataire: **Martin, Jean-Jacques et al, Cabinet
REGIMBEAU 26, Avenue Kléber, F-75116 Paris (FR)**

## Description

La présente invention concerne un matériau chirurgical composite absorbable, un procédé de préparation d'un tel matériau, des prothèses résorbables réalisées à partir dudit matériau, ainsi que l'utilisation de telles prothèses pour la réparation des tissus et/ou des organes.

Le brevet GB 1 008 193 délivré en 1965, décrit des implants chirurgicaux non resorbables. Ils comportent un tissu non résorbable et du collagène.

Au cours des dernières décennies, des progrès remarquables ont été faits en chirurgie humaine et vétérinaire. Ceci est principalement dû au développement de matières nouvelles mieux adaptées aux besoins spécifiques de cette discipline, ainsi qu'aux techniques chirurgicales elles-mêmes utilisant ces matières avec succès.

Dans le domaine de la chirurgie réparatrice qui nous interésse plus particulièrement, on a pu observer des améliorations notoires relatives entre autres aux matériaux dits absorbables ou résorbables.

De tels matériaux résorbables ont déjà été décrits.

Ainsi, dans le domaine de l'osthéosynthèse, la demande de brevet européen 0 011 528 concerne des pièces composites résorbables constituées d'une matrice en polyacide lactique renforcé par des éléments de renforcement à base d'acide polyglycolique. Par moulage sous pression, on réalise de vis, clous, plaques d'osthéosynthèse, ou encore des blocs solides à usiner.

On entend par «absorbables» ou «résorbables» des matériaux qui sont progressivement dégradés lorsqu'ils sont introduits dans le corps humain ou animal. Cette dégradation résulte de l'hydrolyse qui s'effectue au contact des tissus vivants en présence des enzymes protéolytiques que ceux-ci contiennent. Dans le texte qui suit, on utilisera indifféremment les termes «absorbable» ou «résorbable».

On entend par chirurgie réparatrice l'ensemble des actes chrirugicaux qui tendent à restituer aux tissus et aux organes lésés, leur emplacement, leurs connexions avec les autres tissus et organes, et leur anatomie façon aussi proche que possible de ceux qu'ils possèdent dans leur état physiologique normal.

Pour atteindre cette réparation optimale, on comprend l'intérêt de disposer d'un matériau absorbable qui assite, voire supplée, pendant un certain temps tout ou partie d'un tissu ou d'un organe endommagé, ledit matériau étant progressivement hydrolysé pour laisser place aux tissus naturels qui, dans le même temps, se seront consolidés et seront en bonne voie de cicatrisation.

Le processus de cicatrisation d'une plaie peut en effet se diviser en trois phases. Un première phase consiste en une phase de latence d'une durée de 3 à 5 jours. Au cours de cette période, les tissus rapprochés et maintenus en place et au contact les uns des autres au moyen de «prothèses» en matériau absorbable, n'établissent aucune connexion entre eux et la plaie ne présente comme résistance que celle donnée par la «prothèse» absorbable. Une seconde phase dite parfois phase de fibroplastie au cours de laquelle les tissus commencent à se souder les uns aux autres, peut s'étendre jusqu'au 10ème jour. Au cours de cette phase, la plaie commence à acquérir une certaine résistance propre qui croît rapidement au cours du temps. Une troisième phase enfin, est une phase de contraction de la cicatrice.

Il apparaît ainsi que les qualités essentielles que devrait posséder un tel matériau, seraient (i) une bonne tolérance par l'organisme, c'est-à-dire n'entraîner aucun phénomène allergique et ne provoquer, ni ne favoriser aucune infection (ii) une bonne résistance mécanqiue, notamment une bonne résistance à la traction, et (iii) une résorption graduelle et lente pendant la phase de latence et le début de la phse de fibroplastie de façon à ce que le matériau absorbable conserve une résistance suffisante par rapport à sa résistance mécanique initiale pendant toute cette période et que les tissus, en voie de cicatrisation, acquièrent une résistance propre leur permettant de prendre le relais du matériau absorbable.

Il convient de préciser que le terme «prothèse» employé dans la présente description désigne de façon très générale tout matériel implanté dans le corps humain et/ou animal, destiné à assister et/ou remplacer temporairement une structure physiologique – tissus, organes – momentanément déficiente. Parmi les prothèses les plus couramment utilisées en chirurgie, on peut citer les fils de ligature, les fils de suture, et d'un usage plus récent les tissus ou tricots résorbables.

L'objet de la présente invention se rapporte plus particulièrement à ces tissus ou tricots que l'on désigne par «treillis» dans le reste de la description. Par «treillis», on entend tout produit textile présentant une structure générale semblable à celle d'une gaze, obtenu par assemblage de fils qui seront avantageusement de même nature que les fils de ligature ou suture habituellement utilisés en chirurgie, l'assemblage de ces fils pouvant être effectué par tout moyen connu tel que tressage, tissage, assemblage par tricot ou crochet, par exemple. De tels treillis trouvent leurs applications entre autres dans le traitement des larges expositions viscérales pour lesquelles ils permettent la contention des organes à vif, dans le traitement conservateur des reins et des rates traumatiques, ou comme substitut du péricarde après certaines exérèses du cancer pulmonaire.

Ces treillis, le plus souvent fabriqués par tissage ou tricottage de fils synthétiques résorbables classiques, présentent des qualités de tolérance par l'organisme, de résistance mécanique et de vitesse de résorption satisfaisantes en général. Cependant leur perméabilité, inhérente à leur structure en gaze et souhaitée dans les applications énoncées ci-avant, les empêchent d'être utilisés pour la réparation de tissus ou d'organes nécessitant le maintien d'une certaine étanchéité. En effet, dans le cas d'une éviscération par exemple, la perméabilité du treillis absorbable mis en place pour contenir efficacement les organes, constitue un atout supplémentaire puisqu'elle permet en plus l'évacuation du sang, pus ou autre sérosité s'écoulant ordinairement d'une plaie «fraichement» soignée. En revanche, si l'on désire utiliser un tel treillis pour la réparation d'une partie d'intestin, d'urètre ou pour réaliser l'anastomose de plusieurs vaisseaux entre eux par exemple, ou des prothèses vasculaires, on comprend la nécessité de disposer d'un matériau résorbable étanche.

C'est pourquoi la présente invention concerne un matériau chirurgical composite absorbable comprenant une armature essentiellement constituée de fils synthétiques résorbables, et une feuille de collagène reconstitué, ladite feuille étant stratifiée avec au moins une face de ladite armature en vue de combler de façon étanche les interstices entre les fils synthétiques résorbables.

Ainsi la feuille de collagène reconstitué qui est adjointe au treillis constitué par l'armature en fils synthétiques résorbables assemblés entre eux de façon appropriée, détermine l'étanchéité du matériau chirurgical. Cette feuille peut également être réalisé en la même matière synthétique résorbable que les fils de l'armature, cependant, on obtient alors un matériau empesé, rigide et cassant. On préfère utiliser du collagène reconstitué, substance naturelle également dégradée par les tissus vivants. Celui-ci est fabriqué à partir du tendon de bœuf qui est surgelé, réduit sous forme de tranches très minces, soumis à un traitement chimique permettant d'obtenir un gel pur et homogène, et reconstitué en fibre solides par coagulation. Le collagène reconstitué, par rapport au collagène – matière première du «CATGUT» et fabriqué à partir de la sous-muqueuse de l'intestin grêle de mouton – constitue une matière de qualité plus homogène déterminant une vitesse d'adsorption uniforme.

Outre le caractère d'étanchéité, la feuille de collagène reconstitué confère un autre avantage au matériau composite. Alors que le treillis seul, du fait qu'il consiste en un produit textile, présente une certaine élasticité, le matériau composite possède une stabilité de forme permettant la réalisation de prothèses tubulées ainsi qu'une manipulation du matériau plus aisée pour le praticien.

De plus, on a découvert de façon surprenante qu'il résultait de la combinaison: feuille de collagène reconstitué/armature en fils synthétiques résorbables, des propriétés biologiques intéressantes de deux ordres: amélioration de la résorbabilité d'une part, meilleure qualité des tissus réparés d'autre part.

L'amélioration de la résorbabilité correspond en fait à un prolongement de celle-ci dans le temps. Ceci résulte du fait que le matériau chirurgical selon l'invention est un matériau composite, c'est-à-dire qu'il est constitué de deux matières d'origine différente: la feuille de collagène reconstitué d'origine naturelle et le treillis d'origine synthétique. On comprend alors aisément qu'une fois au contact des tissus vivants, on observe une hydrolyse différentielle de chacun des deux constituants du matériau composite. Il semble que l'hydrolyse du collagène reconstitué soit la première à se produire. L'hydrolyse des fils synthétiques intervient en second lieu, mais une fois qu'elle a débuté, elle se déroule très rapidement. En revanche, l'hydrolyse du collagène reconstitué est beaucoup plus lente et se poursuit bien après la dégradation totale des fils synthétiques. La combinaison selon l'invention permet donc de disposer d'un matériau chirurgical absorbable se dégradant graduellement et présentant ainsi une résistance mécanique suffisamment importante, en pourcentage de résistance mécanique initiale, pendant toute la phase de latence et la phase de fibroplastie. On sait en effet que le collagène possède une résistance à la traction exceptionnelle. A la fin de la phase de fibroplastie, les tissus corporels commencent à se cicatriser et prennent progressivement le relais du matériau chirurgical dont la dégradation se poursuit.

Enfin, en comparant les tissus réparés contre un matériau synthétique et les tissus réparés contre un matériau composite conforme à la présente invention, on constate que la qualité de cas tissus est nettement meilleure dans la deuxième hypothèse. En effet, lorsqu'on pose une prothèse en un matériau uniquement synthétique sur une plaie, on observe que les tissus une fois cicatrisés présentent des zones de réctraction. On parle également de «perte de matière». Ces phénomènes ne sont pas observés avec une prothèse en un matériau composite selon l'invention. Ceci est principalement dû à la présence du collagène reconstitué dont la nature particulière suscite la fibrose. Il se forme un tissu riche en fibres n'entraînant pas de phénomènes de réctraction.

Selon un mode de réalisation de l'invention, l'armature du matériau chirurgical consiste en un treillis de fils synthétiques résorbables à base d'un polymère de l'acide glycolique, encore appelé acide hydroxy-acétique. Les esters polyhydroxy-acétiques ont été décrits entre autres dans les brevets américains n° 2 668 162, n° 2 758 987 et le brevet français n° 1 412 957.

Selon un mode de réalisation préféré de l'invention, les fils synthétiques résorbables dudit treillis sont en un copolymère d'acide glycolique et d'acide lactique. On utilisera de préférence un treillis VICRYL®-polyglactine 910 qui est un copolymère comportant environ 90% d'acide glycolique et environ 10% d'acide lactique.

La présente invention concerne également un procédé de préparation du matériau chirurgical composite absorbable en question. Ce procédé comprend les étapes suivantes:

a) assemblage des fils synthétiques résorbables de façon appropriée pour confectionner l'armature désirée,,

b) préparation d'une dispersion de collagène reconstitué dans un solvant approprié,

c) stratification de l'armature confectionnée en a) avec le collagène reconstitué, en enduisant au moins une des faces de ladite armature avec la dispersion de collagène reconstitué préparée en b),

d) et dessication du matériau composite ainsi obtenu.

Selon un mode de réalisation particulier de l'invention, le procédé comprend les étapes suivantes:

a) confection d'un treillis de fils résorbables synthétiques en un copolymère d'acide glycolique et d'acide lactique,

b) préparation d'un gel de collagène reconstitué dans un solvant approprié,

c) stratification du treillis confectionné en a) avec le collagène reconstitué à l'état de gel, en disposant ledit treillis sur une surface nan adhésive, en coulant sur les treillis une mince couche dudit gel, en laissant tremper,

d) et en laissant sécher.

Selon un autre mode particulier de réalisation de l'invention, le procédé comprend les étapes suivantes:

a) confection d'un treillis de fils résorbables synthétiques en un copolymère d'acide glycolique et d'acide lactique,

b) préparation d'un gel de collagène reconstitué dans un solvant approprié,

c) stratification du treillis confectionné en a) avec le collagène reconstitué à l'état de gel, en étalant une mince couche dudit gel sur une surface non adhésive, en déposant sur cette couche ledit treillis, en laissant tremper,

d) et en laissant sécher.

De préférence, on utilisera un treillis VICRYL®-polyglactine 910 dont les fils constitués d'un copolymère d'acide glycolique et d'acide lactique, sont tricotés selon un procédé spécial dit «interlock» évitant le démaillage. La taille des mailles peut varier. On pourra employer notamment une maille fine (n° 9) ou une résille plus large (n° 7).

De préférence, le solvant utilisé pour la préparation du gel de collagène reconstitué est de l'eau.

Comme surface non adhésive, on peut utiliser une plaque de verre ou de métal ou de matière synthétique non adhésive; on emploie préférentiellement une plaque de verre ou de plastique.

Enfin, la stratification décrite ici a lieu sur au moins une des faces de l'armature. Ceci signifie que le treillis qui présente une certaine épaisseur peut être stratifié sur une fraction de cette épaisseur ou sur la totalité de celle-ci – en recouvrant par exemple, à l'étape c), le treillis d'une couche supplémentaire de gel de collagène reconstitué.

Un autre objet de la présente invention consiste en une prothèse résorbable pour le renforcement temporaire des tissus du corps humain et/ou animal, réalisée à partir du matériau chirurgical composite décrit. Compte-tenu des propriétés particulières tant mécaniques: étanchéité, stabilité de forme, que biologique: néogénèse des tissus sans perte de matière, que présente ledit matériau, on réalisera de façon avantageuse des prothèses tubulées.

Ainsi, les applications préférées d'une prothèse selon l'invention consistent en leur utilisation pour la réparation de tissus ou d'organes, notamment pour la réparation d'organes ayant une section tubulaire.

Comme cela apparaîtra dans les exemples, on a pu montré l'efficacité des treillis selon l'invention pour des prothèses et la tolérance tissulaire des matériaux selon l'invention.

Parmi les utilisations préférées, on peut citer:
– la réparation de tout ou partie de l'appareil digestif, notamment tout ou partie du duodénum, du canal cholédoque ou d'intestins,
– la réparation de tout ou partie de l'appareil urinaire, notamment tout ou partie d'uretères, d'urètre,
– l'anastomose de deux ou plusieurs vaisseaux sanguins entre eux,
– la réalisation de prothèses vasculaires.

D'autres caractéristiques et avantages de l'invention apparaîtront à la lecture des exemples et de la description détaillée qui va suivre, en se référant aux dessins annexés. Sur ces dessins:

– les figures 1 et 2 représentent à l'échelle agrandie, deux exemples de treillis VICRYL® polyglactine 910 utilisables comme armature pour le matériau selon l'invention,

– la figure 3 représente une coupe schématique de la structure stratifiée d'un matériau chirurgical composite absorbable selon l'invention réalisé avec une armature telle que le treillis représenté à la figure 2, et selon AA représenté sur cette figure 2.

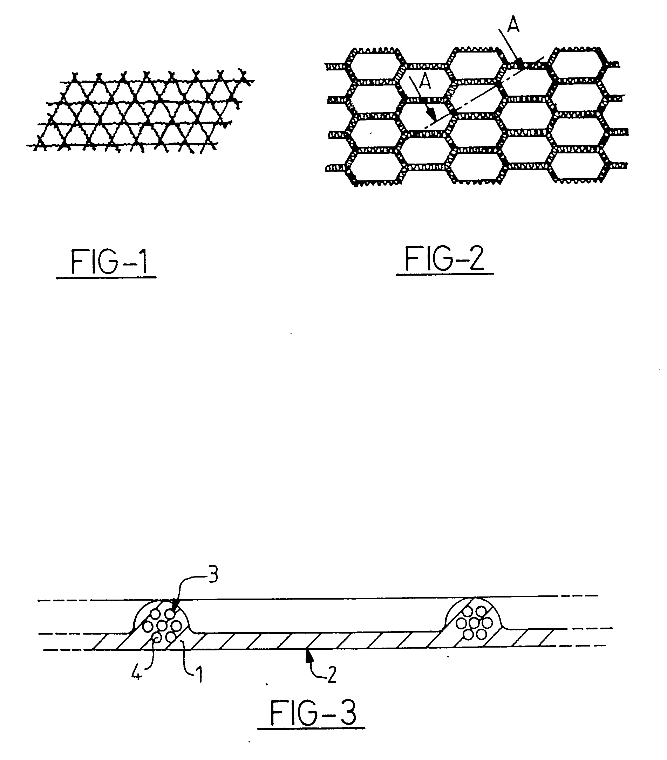

La figure 1 représente les mailles d'un treillis de VICRYL® polyglactine 910 (maille 9) utilisé comme armature. Les mailles de forme sensiblement triangulaires, d'environ 0,5 mm de côté sont disposées en rangées parallèles, chacune desdites rangées étant constitué de mailles en organisation alternée.

La figure 2 représente les mailles d'un treillis VICRYL® polyglactine 910 (maille 7) utilisé comme armature. Les mailles hexagonales de dimension moyenne $2,5 \times 1,7$ en mm constituent un système hexagonal.

Comme on le voit sur la figure 3, le nouveau matériau chirurgical selon l'invention est constitué d'un treillis 3 et d'une feuille de collagène reconstitué 1. Le collagène reconstitué 1, représenté par les hachures noie en imprégnant les fils 4 du treillis 3 solidaire de la feuille 2.

Pour réaliser un tel matériau composite, on dispose sur une plaque de verre le treillis 3 et on coule par dessus un gel aqueux de collagène reconstitué. Le treillis 3 s'imprègne alors de collagène reconstitué qui noie les fils 4, dont le nombre représenté est arbitraire, et bouche les trous laissés par les mailles du treillis 3 en formant une feuille 2 solidaire du treillis 3 imprégné. Le treillis se trouve ainsi comblé sur une fraction de son épaisseur. Dans un courant d'air, on déshydrate alors le matériau composite obtenu qui se sépare de la plaque de verre.

La description précédente ne constitue qu'un exemple particulier de mise en œuvre du procédé et de réalisation de l'invention, et ne saurait limiter la portée de celle-ci qui englobe toute variante reprenant des moyens équivalents.

Exemple 1:

Procédé de préparation du treillis de VICRYL® polyglactine stratifié avec du collagène.

A) On prépare une suspension de collagène, provenant de cuir de bœuf, contenant au plus 0,5% en poids de collagène, dans une solution acide de 0,05 m d'acide acétique, à 4°C.

B) La suspension est homogénéisée dans un mélangeur classique jusqu'à ce qu'elle atteigne une turbidité constante, ce que l'ont peut contrôler en spectrophotométrie à 440 nm par exemple.

C) La suspension est dégazée sous vide.

D) On verse alors la suspension sur un plateau en plastique – et on la laisse sécher à température ordinaire sous courant d'air filtré. Il se forme un feuille de collagène.

E) On dispose à plat sur la feuille ainsi préparée un treillis VICRYL® polyglactine.

F) On verse sur le treillis disposé sur la feuille une suspension de collagène telle que préparée dans les étapes A à C. L'air piégé est chassé et le tout est séché sous courant d'air filtre.

G) Le treillis VICRYL®-collagène ainsi préparé est retiré du plateau plastique et scellé sous feuille de métal.

Ce mode de préparation n'est décrit qu'à titre d'exemple, d'autres variantes sont envisageables. Ainsi, des concentrations plus fortes en collagène peuvent être utilisées, d'autres sources de collagène conviennent également; tout autre acide minéral ou organique, pourvu qu'il ne soit ni toxique, ni néfaste pour le produit final peut être employé. La solution acide peut en outre contenir un plastifiant, par exemple de glycérol.

Exemple 2:

Les treillis selon l'invention ont fait l'objet de tests in vivo de tolérance tissulaire. Des études anatomopathologiques ont été menées.

· Sur le chien, en particulier sur la paroi stomacale, la vésicule biliaire et l'oesophage (reconstruction de l'oesophage).

On assiste en deux mois à la régénération complète de l'épithélium, à la reconstitution presque totale de la musculeuse et, en cas de plastie tubulaire (oesophage 5 cm), à une reconstitution des trois tuniques avec une sténose modérée (Etude réalisée à l'hôpital BEAUJON (CLICHY, France).

· Sur le rat.

Après une implantation dans le tissu cellulaire souscutané, on note une réaction tissulaire nette au 7è jour suivie d'un processus normal de résorption avec excellente tolérance tissulaire (Etude réalisée à INSERM (LYON, France).

**Revendications**

1. Matériau chirurgical composite absorbable comprenant une armature essentiellement constituée de fils synthétiques résorbables, et une feuille de collagène reconstitué, ladite feuille étant stratifiée avec au moins une face de ladite armature en vue de combler de façon étanche les interstices entre les fils synthétiques résorbables.

2. Matériau chrirugical selon la revendication 1, caractérisé en ce que ladite armature consiste en un treillis de fils synthétiques résorbables à base d'un polymère de l'acide glycolique.

3. Matériau chirurgical selon la revendication 2, caractérisé en ce que les fils synthétiques résorbables dudit treillis sont en un copolymère d'acide glycolique et d'acide lactique.

4. Matériau chirurgical selon la revendication 3, caractérisé en ce que ledit treillis est un treillis VICRYL®-polyglactine 910.

5. Procédé de préparation d'un matériau chirurgical selon la revendication 1, caractérisé en ce qu'il comprend les étapes suivantes:

a) assemblage des fils synthétiques résorbables de façon appropriée pour confectionner l'armature désirée,

b) préparation d'une dispersion de collagène reconstitué dans un solvant approprié,

c) stratification de l'armature confectionnée en a) avec le collagène reconstitué, en enduisant au moins une des faces de ladite armature avec la dispersion de collagène reconstitué préparée en b),

d) et dessication du matériau composite ainsi obtenu.

6. Procédé selon la revendication 5, caractérisé en ce qu'il comprend les étapes suivantes:

a) confection d'un treillis de fils résorbables synthétiques en un copolymère d'acide glycolique et d'acide lactique,

b) préparation d'un gel de collagène reconstitué dans un solvant approprié,

c) stratification du treillis confectionné en a) avec le collagène reconstitué à l'état de gel, en disposant ledit treillis sur une surface non adhésive, en coulant sur le treillis une mince couche dudit gel, en laissant tremper,

d) et dessication du matériau composite obtenu.

7. Procédé selon la revendication 5, caractérisé en ce qu'il comporte les étapes suivantes:

a) confection d'un treillis de fils résorbables synthétiques en un copolymère d'acide glycolique et d'acide lactique,

b) préparation d'un gel de collagène reconstitué dans un solvant approprié,

c) stratification du traillis confectionné en a) avec le collagène reconstitué à l'état de gel, en étalant une mince couche dudit gel sur une surface non adhésive, en déposant sur cette couche ledit treillis, en laissant tremper,

d) et dessication du matériau composite obtenu.

8. Procédé selon la revendication 5, caractérisé en ce qu'il comporte les étapes suivantes:

a) assemblage des fils synthétiques résorbables de façon appropriée pour confectionner l'armature désirée,

b) préparation d'une suspension de collagène reconstitué dans un solvant approprié,

c) confection d'une feuille de collagène en coulant sur une surface non adhésive une couche de la suspension préparée en h), et en laissant sécher,

d) stratification du treillis confectionné en a) avec le collagène préparé en b) en disposant sur la feuille de collagène confectionné en c) ledit treillis et en coulant sur le treillis une couche de la suspension préparée en b), et

e) dessication du matériau composite obtenu.

9. Prothèse résorbable pour le renforcement temporaire des tissus du corps humain et/ou animal, réalisée à partir du matériau chirurgical selon l'une des revendications précédentes.

10. Utilisation d'une prothèse selon la revendication 9 pour la réparation de tissus et d'organes.

11. Utilisation d'une prothèse selon la revendication 10 pour la réparation d'organes de section tubulaire.

12. Utilisation d'une prothèse selon la revendication 11 pour le réparation de tout ou partie de l'appareil digestif, notamment tout ou partie du duodénum, du canal cholédoque ou des intestins.

13. Utilisation d'une prothèse selon la revendication 11 pour la réparation de tout ou partie de l'appareil urinaire, notamment tout ou partie d'uretères ou d'urètre.

14. Utilisation d'une prothèse selon la revendication 11 pour réaliser l'anastomose de deux ou plusieurs vaisseaux sanguins entre eux.

15. Utilisation d'une prothèse selon la revendication 11 pour la réparation de vaisseaux sanguins.

**Patentansprüche**

1. Absorbierbares chirurgisches Verbundmaterial mit einer im wesentlichen aus resorbierbaren synthetischen Fäden gebildeten Einlage und einer Folie aus rekonstituiertem Kollagen, wobei die Folie mit wenigstens einer Fläche der Einlage geschichtet ist, um die Zwischenräume zwischen den resorbierbaren synthetischen Fäden dicht zu verschließen.

2. Chirurgisches Material nach Anspruch 1, dadurch gekennzeichnet, daß die Einlage aus einem Netzwerk von resorbierbaren synthetischen Fäden auf der Basis eines Glykolsäure-Polymers besteht.

3. Chirurgisches Material nach Anspruch 2, dadurch gekennzeichnet, daß die resorbierbaren synthetischen Fäden des Netzwerks aus einem Copolymer von Glykolsäure und Milchsäure sind.

4. Chirurgisches Material nach Anspruch 3, dadurch gekennzeichnet, daß das Netzwerk ein VI-CRYL®-Polyglactin 910-Netzwerk ist.

5. Verfahren zur Herstellung eines chirurgischen Materials nach Anspruch 1, dadurch gekennzeichnet, daß es die folgenden Schritte umfaßt:

a) Verbinden der resorbierbaren synthetischen Fäden in zweckdienlicher Weise zur Konfektionierung der gewünschten Einlage,

b) Herstellen einer Dispersion von rekonstituiertem Kollagen in einem zweckdienlichen Lösungsmittel,

c) Schichten der in (a) konfektionierten Einlage mit dem rekonstituierten Kollagen durch Überziehen wenigstens einer der Flächen der Einlage mit der in (b) hergestellten Dispersion von rekonstituiertem Kollagen,

d) und Tocknen des so erhaltenen Verbundmaterials.

6. Verfahren nach Anspruch 5, dadurch gekennzeichnet, daß es die folgenden Schritte umfaßt:

a) Konfektionieren eines Netzwerks von synthetischen resorbierbaren Fäden aus einem Copolymer von Glykolsäure und Milchsäure,

b) Herstellen eines Gels aus rekonstituiertem Kollagen in einem zweckdienlichen Lösungsmittel,

c) Schichten des in (a) konfektionierten Netzwerks mit dem im Gelzustand befindlichen rekonstituierten Kollagen durch Anordnen des Netzwerks auf einer nicht klebenden Fläche, Aufgießen einer dünnen Schicht des Gels auf das Netzwerk, Einziehenlassen,

d) und Trocknen des erhaltenen Verbundmaterials.

7. Verfahren nach Anspruch 5, dadurch gekennzeichnet, daß es folgende Schritte umfaßt:

a) Konfektionieren eines Netzwerks von synthetischen resorbierbaren Fäden aus einem Copolymer von Glykolsäure und Milchsäure,

b) Herstellen eines Gels aus rekonstituiertem Kollagen in einem zweckdienlichen Lösungsmittel,

c) Schichten des in (a) konfektionierten Netzwerks mit dem im Gelzustand befindlichen rekonstituierten Kollagen durch Auftragen einer dünnen Schicht des Gels auf eine nicht klebende Fläche, Auflegen des Netzwerks auf diese Schicht, Einziehenlassen,

d) und Trocknen des erhaltenen Verbundmaterials.

8. Verfahren nach Anspruch 5, dadurch gekennzeichnet, daß es die folgenden Schritte umfaßt:

a) Verbinden der resorbierbaren synthetischen Fäden in zweckdienlicher Weise zum Konfektionieren der gewünschten Einlage,

b) Herstellen einer Suspension von rekonstituiertem Kollagen in einem zweckdienlichen Lösungsmittel,

c) Konfektionieren einer Kollagen-Folie durch Aufgießen einer Schicht in (h) hergestellter Suspension auf eine nicht klebende Fläche, und Trocknenlassen,

d) Schichten des in (a) konfektionierten Netzwerks mit dem in (b) hergestellten Kollagen durch Auflegen des Netzwerks auf die in (c) konfektionierte Kollagen-Folie und Aufgießen einer Schicht in (b) hergestellter Suspension auf das Netzwerk, und

e) Trocknen des erhaltenen Verbundmaterials.

9. Resorbierbare Prothese zur vorübergehenden Verstärkung menschlichen und/oder tierischen Körpergewebes, hergestellt aus dem chirurgischen Material gemäß einem der vorhergehenden Ansprüche.

10. Benutzung einer Prothese nach Anspruch 9, zur Wiederherstellung von Geweben und Organen.

11. Benutzung einer Prothese nach Anspruch 10, zur Wiederherstellung von Organen rohrförmigen Querschnitts.

12. Benutzung einer Prothese nach Anspruch 11 zur Wiederherstellung von Teilen oder des gesamten Verdauungstraktes, insbesondere von Teilen oder des gesamten Duodenums, Ausführungsgangs der Leber oder der Eingeweide.

13. Benutzung einer Prothese nach Anspruch 11 zur Wiederherstellung von Teilen der gesamten Harnwege, insbesondere der Ureter oder der Urethra ganz oder zum Teil.

14. Benutzung einer Prothese nach Anspruch 11 zur Durchführung der Anastomose von zwei oder mehreren Blutgefäßen miteinander.

15. Benutzung einer Prothese nach Anspruch 11 zur Wiederherstellung von Blutgefäßen.

**Claims**

1. Absorbable composite surgical material comprising a reinforcement essentially constituted of resorbable synthetic threads, and a sheet of reconstituted collagen, the said sheet being laminated with at least one face of the said reinforcement for the purpose of filling in a leakproof manner the interstices between the said resorbable synthetic threads.

2. Surgical material as claimed in claim 1 in which the said meshwork consists in resorbable synthetic threads based on a polymer of glycolic acid.

3. Surgical material as claimed in claim 2, in which the resorbable synthetic threads of the said meshwork are mad of a copolymer of glycolic acid and lactic acid.

4. Surgical material as claimed in claim 3, in which the said meshwork is a VICRYL® polyglactine 910 meshwork.

5. Process for preparing a surgical material as claimed in claim 1, which process comprises the following stages:

a) assembly of the resorbable synthetic threads in a suitable manner to form the desired reinforcement,

b) preparation of a dispersion of reconstituted collagen in a suitable solvent,

c) lamination of the reinforcement formed in a) with the reconstituted collagen, by coating at least one of the faces of the said reinforcement with the dispersion of reconstituted collagen prepared in b), and

d) drying of the composite material thereby obtained.

6. Process as claimed in claim 5, which process comprises the following stages:

a) formation of a meshwork of synthetic resorbable threads made of a copolymer of glycolic acid and lactic acid,

b) preparation of a gel of reconstituted collagen in a suitable solvent,

c) lamination of the meshwork formed in a) with the reconstituted collagen in the gel state, by arranging the said meshwork on a non-adhesive surface, pouring a thin layer of the said gel onto the meshwork, leaving the materiel so soak, and

d) drying of the composite material obtained.

7. Process as claimed in claim 5, which process comprises the following stages:

a) formation of a meshwork of synthetic resorbable threads made of a copolymer of glycolic acid and lactic acid,

b) preparation of a gel of reconstituted collagen in a suitable solvent,

c) lamination of the meshwork formed in a) with the reconstituted collagen in the gel state, by spreading a thin layer of the said gel on a non-adhesive surface, depositing on this layer the said meshwork, leaving the material to soak, and

d) drying of the composite material obtained.

8. Process is claimed in claim 5, which process comprises the following stages:

a) assembly of the resorbable synthetic threads in a suitable manner to form the desired reinforcement

b) preparation of a suspension of reconstituted collagen in a suitable solvent,

c) formation of a sheet of collagen by pouring a layer of the suspension prepared in b) onto a non-adhesive surface, and allowing this to dry,

d) lamination of the meshwork formed in a) with the collagen prepared in b), by arranging the said meshwork on the sheet of collagen formed in c) and pouring a layer of the suspension prepared in b) onto the meshwork, and

e) drying of the composite material obtained.

9. Resorbable prosthesis for the temporary reinforcement of tissues of the human and/or animal body, produced from the surgical material as claimed in one of the preceding claims.

10. Use of a prosthesis as claimed in claim 9, for repairing tissues and organs.

11. Use of a prosthesis as claimed in claim 10 for repairing organs of tubular section.

12. Use of a prosthesis as claimed in claim 11 for repairing all or part of the digestive system, in particular all or part of the duodenum, bile duct or intestine.

13. Use of a prosthesis as claimed in claim 11 for repairing all or part of the urinary system, in particular all or part of the ureters or the urethra.

14. Use of a prosthesis as claimed in claim 11 for forming an anastomosis of two or more blood vessels with each other.

15. Use of a prosthesis as claimed in claim 1 for repairing blood vessels.

FIG-1

FIG-2

FIG-3